# EUROPEAN PATENT APPLICATION

(11) **EP 4 201 296 A1**
(43) Date of publication of application: **28.06.2023**
(21) Application number: 21857628.8
(22) Date of filing: 16.08.2021
(51) Int. Cl.: A61B 1/00

(54) **IMAGE-BASED POSITION DETECTION METHOD, ELECTRONIC DEVICE, AND READABLE STORAGE MEDIUM**

(30) Priority: 18.08.2020 CN 202010828250
(71) Applicant: Ankon Technologies Co., Ltd, Wuhan, Hubei 430000 (CN)
(72) Inventor: YANGDAI, Tianyi, Wuhan, Hubei 430000 (CN); YUAN, Wenjin, Wuhan, Hubei 430000 (CN); LI, Yi, Wuhan, Hubei 430000 (CN); LIU, Hao, Wuhan, Hubei 430000 (CN)
(74) Representative: Lavoix
(86) International application number: PCT/CN2021/112776
(87) International publication number: WO 2022/037534

(57) **Abstract**

The present invention provides an image-based position detection method, an electronic device, and a readable storage medium. The method includes: driving a capsule endoscope to continuously capture a group of images at an interval of a first preset time t1, wherein each group of images comprises at least one image; obtaining at least one image from each group of images, and calculating a determination index according to grayscale values of at least two channels of the image; determining whether the capsule endoscope reaches a preset detection position according to a value of the determination index; if yes, adjusting the capsule endoscope to a corresponding working mode according to a current position of the capsule endoscope, and if no, continue monitoring. The present invention can determine whether the capsule endoscope reaches the preset detection position, which greatly save battery power, and improve work efficiency of the capsule endoscope.

## Description

### CROSS-REFERENCE TO RELATED APPLICATION

The application claims priority from Chinese Patent Application No. 202010828250.X, filed August 18, 2020, entitled "Image-Based Position Detection Method, Electronic Device, and Readable Storage Medium", all of which are incorporated herein by reference in their entirety.

### TECHNICAL FIELD

The present invention relates to the field of medical equipment imaging, and more particularly to an image-based position detection method, an electronic device and a readable storage medium.

### BACKGROUND

Capsule endoscopy for gastrointestinal examination is increasingly used. A capsule endoscope is taken orally, then passes through mouth, esophagus, stomach, small intestine, large intestine and is finally excreted. Usually, the capsule endoscope travels passively with the peristalsis of digestive tract, and takes images at a certain frame rate during this process, so that a doctor can check the conditions of regions of the digestive tract of a subject.

Since the capsule endoscope is powered by a built-in battery, its working time is limited, e.g., 8-12h. Meanwhile, the working time is also affected by the frame rate. Generally, the capsule endoscope can take 30000-50000 images. The higher frame rate the capsule endoscope has, the higher detection rate (not easy to miss) can be achieved, but the total working time may be shorter, resulting in a decrease in the completion rate of examination.

The existing capsule endoscopes are generally divided into stomach capsules, small bowel capsules and colon capsules. For the capsule for examination of lower digestive tract, it is very important to save power in the early stage. If the capsule stays in the upper digestive tract, especially in the stomach, for too long, it consumes too much power, which may eventually lead to incomplete examination of the colon, etc.

In the prior art, in order to save power, taking a capsule endoscope for examination of small intestine as an example, the capsule endoscope enters a sleep state after working for 3min after starting up, and wakes up after a fixed period of sleep time (such as 1h45min), or is awaken manually to continue working. In this way, the fixed sleep time is statistically significant because most capsules are in the small intestine or at least not in the colon at 1h45min after ingestion, so this can save some power. However, due to the great difference between different subjects, this mode cannot accurately control the switching time of working mode, and the working efficiency needs to be improved.

### SUMMARY

To solve the problems, the present invention provides an image-based position detection method, an electronic device, and a readable storage medium.

It is an object of present invention to provide an image-based position detection method, comprising: driving a capsule endoscope to continuously capture a group of images at an interval of a first preset time t1, wherein each group of images comprises at least one image;
obtaining at least one image from each group of images, and calculating a determination index according to grayscale values of at least two channels of the image;
determining whether the capsule endoscope reaches a preset detection position according to a value of the determination index;
when the capsule endoscope reaches the preset detection position, adjusting the capsule endoscope to a corresponding working mode according to a current position of the capsule endoscope; and
when the capsule endoscope has does not reach the preset detection position, continue monitoring.

In an embodiment of the present invention, each group of images comprises at least 2 images.

In an embodiment of the present invention, obtaining at least one image from each group of images comprises:
obtaining one image at a last capture position from each group of images.

In an embodiment of the present invention, obtaining at least one image from each group of images comprises:
obtaining images sequentially forward from a last capture position in each group of images, wherein the number of the obtained images in each group of images is ensured to be the same, and the number of the obtained images is at least 2.

In an embodiment of the present invention, obtaining at least one image from each group of images, and calculating the determination index according to the grayscale values of at least two channels of the image comprises:
for each group of images, if the number of the obtained images is 1, grayscale value statistics of a G channel and a B channel of the current image are directly used as a G channel value Gₘₑₐₙ and a B channel value Bₘₑₐₙ required for calculating the determination index, wherein the grayscale value statistic is a mean value of the grayscale value of a corresponding channel in a single image;
if the number of the obtained images is at least 2, a mean value of the grayscale value statistics of the G channel of the images obtained from each group of images is used as the G channel value Gₘₑₐₙ required for calculating the determination index, and a mean value of the grayscale value statistics of the B channel of the images obtained from each group of images are used as the B channel value Bₘₑₐₙ required for calculating the determination index;
wherein the determination index corresponding to each group of images is represented by Y, and Y=Gₘₑₐₙ-k×Bₘₑₐₙ-d, wherein k and d are constants.

In an embodiment of the present invention, determining whether the capsule endoscope reaches the preset detection position according to the value of the determination index comprises:
if the determination index is greater than a preset first index threshold, determining that the capsule endoscope reaches the preset detection position.

In an embodiment of the present invention, the method further comprises: configuring a counter;
obtaining the corresponding determination index immediately after each group of images is generated; if a value of the counter N is greater than or equal to Nₘᵢₙ, determining that the capsule endoscope reaches the preset detection position;
wherein the value of Nₘᵢₙ is a positive integer greater than 1;
wherein the value of the counter is 0 in an initial state;
when the determination index is less than the preset first index threshold and greater than a preset second index threshold, accumulating the value of the counter by 1;
when the determination index is not greater than the preset second index threshold, clearing the value of the counter to zero.

In an embodiment of the present invention, before determining whether the capsule endoscope reaches the preset detection position according to the value of the determination index, the method further comprises:
determining whether an operation time of the capsule endoscope is greater than a preset time threshold, wherein
when the operation time is greater than the preset time threshold, determining whether the capsule endoscope reaches the preset detection position according to the value of the determination index;
when the operation time is not greater than the preset time threshold, determining whether the capsule endoscope reaches the preset detection position according to the value of the determination index after determining that the operation time of the capsule endoscope is greater than the preset time threshold.

In an embodiment of the present invention, in the process of obtaining at least one image from each group of images, the method further comprises:
at least obtaining the exposure parameters corresponding to the images for calculating the determination index;
and determining whether the capsule endoscope reaches the preset detection position according to the value of the determination index and the exposure parameters, wherein the exposure parameters are shutter time and image sensor gain participating in exposure in the process of obtaining the images.

In an embodiment of the present invention, the method further comprises:
configuring a counter;
obtaining the corresponding determination index immediately after each group of images is generated;
if the value of the counter N is greater than or equal to Nₘᵢₙ, determining that the capsule endoscope reaches the preset detection position;
wherein the value of Nₘᵢₙ is a positive integer greater than 1;
wherein the value of the counter is 0 in an initial state;
when the determination index is less than the preset first index threshold and greater than the preset second index threshold, and when time*gain is less than or equal to tg, accumulating the value of the counter by 1;
when the determination index is not greater than the preset second index threshold, and/or when time*gain is greater than tg, clearing the value of the counter to zero;
wherein, tg is a preset check value for exposure parameters, which is a constant;
if the number of the images obtained from each group of images is 1, "time" represents shutter time corresponding to the currently obtained image when exposed, and "gain" represents image sensor gain corresponding to the currently obtained image when exposed;
if the number of the images obtained from each group of images is at least 2, "time" represents a mean value of the shutter time corresponding to the images used for calculating the determination index in each group of images when exposed, and "gain" represents a mean value of the image sensor gain corresponding to the images used for calculating the determination index in each group of images when exposed.

It is another object of the present invention to provide an electronic device, comprising a memory and a processor, wherein the memory stores a computer program that can run on the processor, and the processor executes the program to implement the steps of the image-based position detection method as described above.

It is still another object of the present invention to provide a computer-readable storage medium for storing a computer program, wherein the computer program is executed by the processor to implement the steps of the image-based position detection method as described above.

Compared with the prior art, the present invention has the advantages. According to the image-based position detection method, the electronic equipment and the readable storage medium, whether the capsule endoscope reaches the preset detection position is determined according to the grayscale value of the obtained images, additional hardware assistance is not needed, the requirement on hardware computing power is low, the battery power is greatly saved, and the work efficiency of the capsule endoscope is improved.

### BRIEF DESCRIPTION OF THE DRAWINGS

FIG. 1 is a flow schematic diagram of an image-based position detection method, in accordance with an embodiment of the present invention.
FIG. 2 is a flow schematic diagram of a specific implementation process of step S3 in FIG. 1.
FIG. 3 is a flow schematic diagram of another specific implementation process of step S3 in FIG. 1.
FIG. 4 is a flow schematic diagram of still another specific implementation process of step S3 in FIG. 1.
FIG. 5 is a flow schematic diagram of a preferred embodiment of implementing step S3 in FIG. 4.

### DETAILED DESCRIPTION

The present invention can be described in detail below with reference to the accompanying drawings and preferred embodiments. However, the embodiments are not intended to limit the present invention, and the structural, method, or functional changes made by those skilled in the art in accordance with the embodiments are included in the scope of the present invention.

A capsule endoscope of the present invention is mainly used for examination of lower digestive tract, for example, for examination of small intestine. Correspondingly, structures of the capsule endoscope are the same as those of the existing capsule endoscope. In a specific embodiment of the present invention, after the capsule endoscope captures an image, the image is transmitted to an external portable data recorder through a wireless transmission unit; after the portable data recorder receives the image, an image processing unit processes the image according to the following method steps, and sends a corresponding command to the capsule endoscope through a control unit based on a processing result, so as to identify the position of the capsule endoscope in the digestive tract and adjust the working state of the capsule endoscope. The capsule endoscope can automatically adjust its frame rate, exposure parameters, etc., according to environmental changes, user requirements and other factors in the process of capturing images, and this part belongs to the prior art and is not further described here.

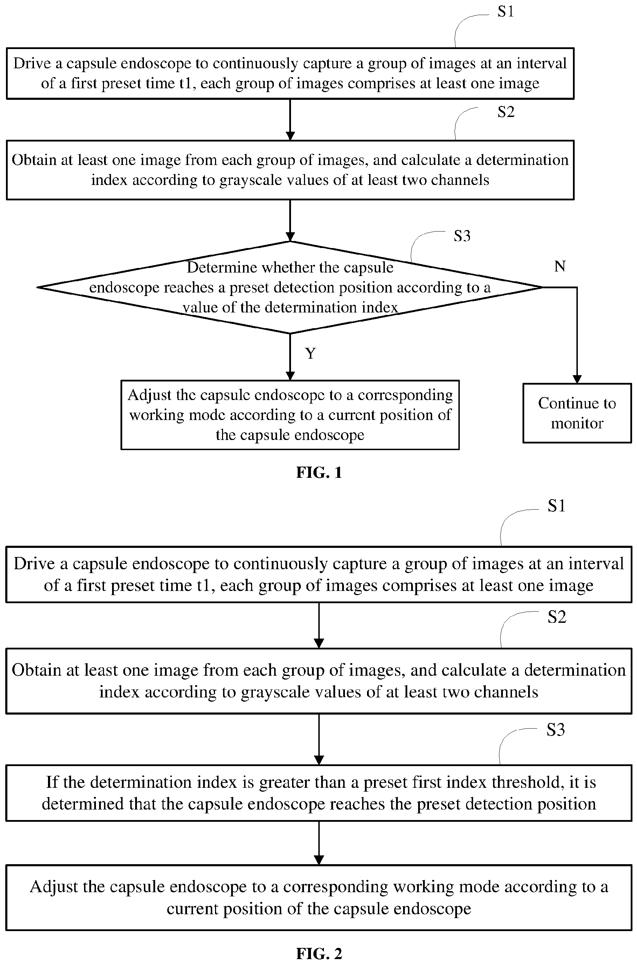

Referring to FIG. 1, in an embodiment of the present invention, an image-based position detection method is provided, the method comprises:
step S1, driving a capsule endoscope to continuously capture a group of images at an interval of a first preset time t1, wherein each group of images comprises at least one image;
step S2, obtaining at least one image from each group of images, and calculating a determination index according to grayscale values of at least two channels of the image;
step S3, determining whether the capsule endoscope reaches a preset detection position according to the value of the determination index;
when the capsule endoscope reaches the preset detection position, adjusting the capsule endoscope to a corresponding working mode according to a current position of the capsule endoscope; and
when the capsule endoscope has does not reach the preset detection position, continue monitoring.

Preferably, before the step S1, images may be captured at a fixed frame rate f0 within a certain time T0, so as to observe the environment before the preset detection position. For example, the preset detection position is the small intestine, and a step before step S1 is used to observe the esophagus, etc. After that, the process proceeds to step S1. In a specific embodiment of the present invention, the value of f0 is: 2-16fps, preferably 4-8fps, and the value of T0 is 3-10min.

In step S1, the capsule endoscope is in a sleep state for most of the time, and a sleep time can be adjusted according to the value of the first preset time t1, so that the current position of the capsule endoscope can be accurately determined in a state of efficient operation of the capsule endoscope.

In a specific embodiment of the present invention, the value of t1 may be set to 100-900s. That is, in step S1, the capsule endoscope is awakened every the time t1, while a set number of images are captured.

Preferably, in step S1, when the capsule endoscope is awakened, a group of images are continuously captured at a fixed frame rate f1, and the value of f1 can be specifically set as required. In a specific embodiment of the present invention, f1 is set to a relatively high value, for example: f1 is set to 2-6fps.

Preferably, the number of images included in each group of images can be set according to actual needs, and the number of images in different groups of images can be the same or different. In a specific embodiment of the present invention, the number of images continuously captured in each group is set to be the same.

Preferably, each group of images comprises at least 2 images. In a specific embodiment of the present invention, the number of images in each group is set to 2-5. Once the images of each group are captured, they are immediately transmitted to the external portable data recorder, and analyzed therein for determining the position of the capsule endoscope.

Due to an AEC (Auto-Exposure Control) of the capsule endoscope takes some time to stabilize (depending on a specific AEC method), usually the first image may have poor exposure (too dark or overexposed). Therefore, each group of images comprises at least 2 images, namely at least 2 images are continuously captured, so that the quality of the images can be ensured, and the calculation accuracy is improved.

In step S2, in a first preferred embodiment of the present invention, one image at a last capture position is obtained from each group of images. Accordingly, the last image in each group of images is obtained for calculation, so that the exposure value of the image is more stable, and the calculation accuracy can be improved.

In step S2, in a second preferred embodiment of the present invention, for each group of images, the images are obtained sequentially forward from the last capture position in each group of images, wherein the number of the obtained images in each group of images is ensured to be the same, and the number of the obtained images is at least 2.

Further, in step S2, each image comprises three channels of grayscale values, namely, R, G, and B channels of grayscale values. In a specific embodiment of the present invention, the grayscale values of at least two channels are selected from the three channels for calculating the determination index.

In a specific embodiment of the present invention, for each image used for calculation, 2 channels of grayscale values are selected, which are the grayscale values of the G channel and the B channel, respectively.

Specifically, for each group of images, if the number of the obtained images is 1, grayscale value statistics of the G channel and the B channel of the current image are directly used as the G channel value Gₘₑₐₙ and the B channel value Bₘₑₐₙ required for calculating the determination index. The grayscale value statistic is a mean value of the grayscale value of a corresponding channel in a single image.

If the number of the obtained images is at least 2, the mean value of the grayscale value statistics of the G channel of the images obtained from each group of images is used as the G channel value Gₘₑₐₙ required for calculating the determination index, and the mean value of the grayscale value statistics of the B channel of the images obtained from each group of images are used as the B channel value Bₘₑₐₙ required for calculating the determination index.

The determination index corresponding to each group of images is represented by Y, and Y=Gₘₑₐₙ-k×Bₘₑₐₙ-d, wherein k and d are constants. In a specific embodiment of the present invention, the value k takes a value around 1 and the value d takes a value around 20.

Referring to FIG. 2, in step S3, in the first preferred embodiment of the present invention, if the determination index is greater than a preset first index threshold, it is determined that the capsule endoscope reaches the preset detection position.

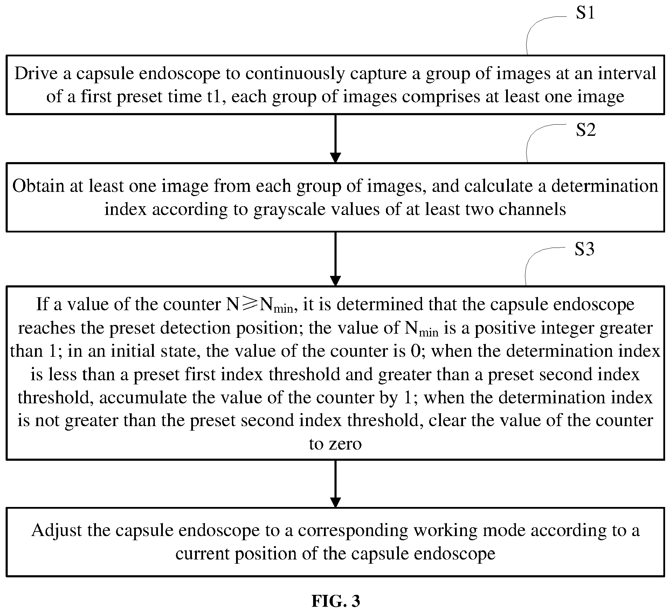

Referring to FIG. 3, in the second preferred embodiment of the present invention, in step S3, configuring a counter, and determining whether the capsule endoscope reaches the preset detection position according to the value of the counter.

Specifically, the corresponding determination index is obtained immediately after each group of images is generated, and if the value of the counter N is greater than or equal to Nₘᵢₙ, it is determined that the capsule endoscope reaches the preset detection position;
wherein the value of Nₘᵢₙ is a positive integer greater than 1;
in an initial state, the value of the counter is 0;
when the determination index is less than the preset first index threshold and greater than a preset second index threshold, the value of the counter is accumulated by 1;
when the determination index is not greater than the preset second index threshold, the value of the counter is cleared to zero.

The value of the counter is adjusted by a plurality of determination indexes obtained by defining a time sequence, and whether the capsule endoscope reaches a preset detection position is determined by the value of the counter. Thus, taking the examination of the small intestine by the capsule endoscope as an example, the influence of environmental factors, such as bile reflux phenomenon, on the determination result can be avoided, and the calculation accuracy is improved.

The preset first index threshold is greater than the preset second index threshold, and the value of the preset first index threshold can be set as required. In a specific embodiment of the present invention, the value of the preset second index threshold is set to 0, and the value of the preset first index threshold belongs to the interval (5, 20), for example, the preset first index threshold is set to 10, and the value of Nₘᵢₙ is set to 2.

Preferably, as for step S3, the present invention provides a third preferred embodiment to implement step S3. In the third preferred embodiment, in the process of determining whether the capsule endoscope reaches the preset detection position, exposure parameters when an image is captured are introduced, so that auxiliary determination parameters for determining whether the capsule endoscope reaches the preset detection position are added, which improves the detection accuracy.

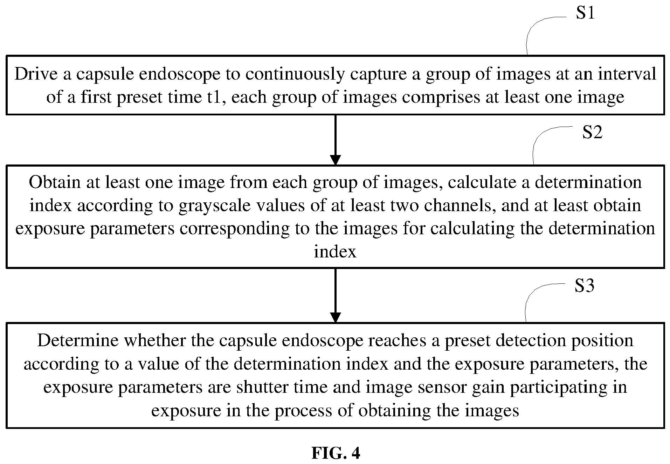

Referring to FIG. 4, specifically, for the third preferred embodiment for implementing step S3, in step S2, it is also required to at least obtain the exposure parameters corresponding to the images for calculating the determination index in the process of obtaining at least one image from each group of images. In step S3, whether the capsule endoscope reaches the preset detection position is determined according to the value of the determination index and the exposure parameters, wherein the exposure parameters comprise shutter time and image sensor gain participating in exposure in the process of obtaining the images.

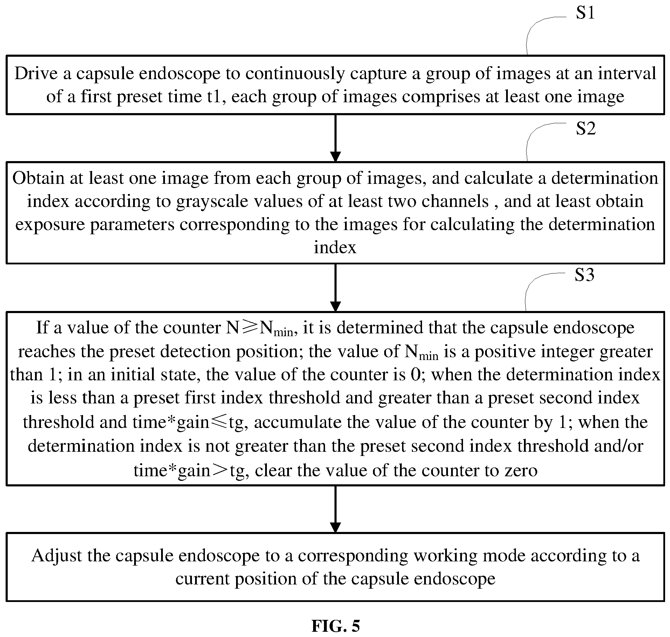

Further, referring to FIG. 5, the third preferred embodiment specifically comprises:
configuring a counter;
obtaining the corresponding determination index immediately after each group of images is generated;
if the value of the counter N is greater than or equal to Nₘᵢₙ, determining that the capsule endoscope reaches the preset detection position;
wherein the value of Nₘᵢₙ is a positive integer greater than 1;
in an initial state, the value of the counter is 0;
when the determination index is less than the preset first index threshold and greater than the preset second index threshold, and when time*gain is less than or equal to tg, accumulating the value of the counter by 1;
when the determination index is not greater than the preset second index threshold, and/or when time*gain is greater than tg, clearing the value of the counter to zero;
wherein tg is a preset check value for exposure parameters and is a constant;
if the number of the images obtained from each group of images is 1, "time" represents the shutter time corresponding to the currently obtained image when exposed, and "gain" represents the image sensor gain corresponding to the currently obtained image when exposed;
if the number of the images obtained from each group of images is at least 2, "time" represents the mean value of the shutter time corresponding to the images used for calculating the determination index in each group of images when exposed, and "gain" represents the mean value of the image sensor gain corresponding to the images used for calculating the determination index in each group of images when exposed.

In the embodiments of the present invention, the exposure parameters are automatically adjusted along with capture environments, so that the brightness of the obtained images is moderate. In a specific embodiment of the present invention, the "time" is generally a value in the range of 0.1-0.8ms, the "gain" is generally a value in the range of 1-8, the tg can be specifically set as required, and the value range of tg in a specific embodiment of the present invention belongs to the interval (0.1ms, 3ms).

In the implementation process of the present invention, the exposure parameters are used as auxiliary parameters for determining whether the capsule endoscope reaches the preset detection position, so that the accuracy can be further improved.

It should be noted that the first preferred embodiment, the second preferred embodiment and the third preferred embodiment for implementing step S3 can be used separately to determine whether the capsule endoscope reaches the preset detection position. Alternatively, the first preferred embodiment and the second preferred embodiment can be used synchronously or sequentially to determine whether the capsule endoscope reaches the preset detection position. Alternatively, the first preferred embodiment and the third preferred embodiment can be used synchronously or sequentially to determine whether the capsule endoscope reaches the preset detection position.

Preferably, before step S3, the method further comprises: determining whether an operation time of the capsule endoscope is greater than a preset time threshold, and if the operation time is greater than the preset time threshold, determining whether the capsule endoscope reaches the preset detection position according to the value of the determination index, and if the operation time is not greater than the preset time threshold, determining whether the capsule endoscope reaches the preset detection position according to the value of the determination index after determining that the operation time of the capsule endoscope is greater than the preset time threshold.

It can be understood that this step can be performed before step S3. However, in order to save the battery power to the greatest extent, this step may be performed before step S1, or step S1 may be performed when the operation time of the capsule endoscope approaches the preset time threshold, which will not be further described herein.

The preset time threshold is also a preset time constant, and its value can be specifically set as required. In the specific embodiments of the present invention, taking the examination of small intestine as an example, the value range of the preset time threshold belongs to the interval (10min, 30min).

Further, after determining that the capsule endoscope reaches the preset detection position, adjusting the working mode of the capsule endoscope accordingly. In this embodiment, after it is determined that the capsule endoscope reaches the small intestine, the working mode of the capsule endoscope is adjusted to be a small intestine detection mode, and in this mode, images are normally captured according to preset conditions for real-time detection, and the specific implementation process is not described here.

Further, it is another object of the present invention to provide an electronic device, comprising a memory and a processor, where the memory stores a computer program that can run on the processor, and the processor executes the program to implement the steps of the image-based position detection method as described above.

Further, it is still another object of the present invention to provide a computer-readable storage medium for storing a computer program, where the computer program is executed by the processor to implement the steps of the image-based position detection method as described above.

To sum up, the image-based position detection method, the electronic device and the readable storage medium of the present invention can simply and efficiently determine whether the capsule endoscope reaches the preset detection position based on the images obtained by the existing hardware structure, without additional hardware assistance, and have low hardware computing power requirement. Meanwhile, in the process of capturing images, an extremely low equivalent frame rate is used, so that the battery power is greatly saved, and the use efficiency of the capsule endoscope is improved.

It should be understood that, although the description is described in terms of embodiments, not every embodiment merely comprises an independent technical solution. Those skilled in the art should have the description as a whole, and the technical solutions in each embodiment may also be combined as appropriate to form other embodiments that can be understood by those skilled in the art.

The series of detailed descriptions set forth above are only specific descriptions of feasible embodiments of the present invention and are not intended to limit the scope of protection of the present invention. Equivalent embodiments or modifications made within the spirit of the present invention shall fall within the protection scope of the present invention.

## Claims

1. An image-based position detection method, comprising:
driving a capsule endoscope to continuously capture a group of images at an interval of a first preset time t1, wherein each group of images comprises at least one image;
obtaining at least one image from each group of images, and calculating a determination index according to grayscale values of at least two channels of the image;
determining whether the capsule endoscope reaches a preset detection position according to a value of the determination index;
adjusting the capsule endoscope to a corresponding working mode according to a current position of the capsule endoscope when the capsule endoscope reaches the preset detection position; and
continue monitoring when the capsule endoscope has does not reach the preset detection position.

2. The image-based position detection method of claim 1, wherein each group of images comprises at least 2 images.

3. The image-based position detection method of claim 2, wherein obtaining at least one image from each group of images comprises:
obtaining one image at a last capture position from each group of images.

4. The image-based position detection method of claim 2, wherein obtaining at least one image from each group of images comprises:
obtaining images sequentially forward from a last capture position in each group of images, wherein the number of the obtained images in each group of images is ensured to be the same, and the number of the obtained images is at least 2.

5. The image-based position detection method of claim 2, wherein obtaining at least one image from each group of images, and calculating the determination index according to the grayscale values of at least two channels of the image comprises:
for each group of images, if the number of the obtained images is 1, grayscale value statistics of a G channel and a B channel of the current image are directly used as a G channel value Gₘₑₐₙ and a B channel value Bₘₑₐₙ required for calculating the determination index, wherein the grayscale value statistic is a mean value of the grayscale value of a corresponding channel in a single image;
if the number of the obtained images is at least 2, a mean value of the grayscale value statistics of the G channel of the images obtained from each group of images is used as the G channel value Gₘₑₐₙ required for calculating the determination index, and a mean value of the grayscale value statistics of the B channel of the images obtained from each group of images are used as the B channel value Bₘₑₐₙ required for calculating the determination index;
wherein the determination index corresponding to each group of images is represented by Y, Y=Gₘₑₐₙ-k×Bₘₑₐₙ-d, and k and d are constants.

6. The image-based position detection method of claim 1, wherein determining whether the capsule endoscope reaches the preset detection position according to the value of the determination index comprises:
determining that the capsule endoscope reaches the preset detection position when the determination index is greater than a preset first index threshold.

7. The image-based position detection method of claim 1, further comprising: configuring a counter;
obtaining the corresponding determination index immediately after each group of images is generated; determining that the capsule endoscope reaches the preset detection position when a value of the counter N is greater than or equal to Nₘᵢₙ;
wherein the value of Nₘᵢₙ is a positive integer greater than 1;
wherein the value of the counter is 0 in an initial state;
accumulating the value of the counter by 1 when the determination index is less than a preset first index threshold and greater than a preset second index threshold;
clearing the value of the counter to zero when the determination index is not greater than the preset second index threshold.

8. The image-based position detection method of claim 1, wherein before determining whether the capsule endoscope reaches the preset detection position according to the value of the determination index, the method further comprises:
determining whether an operation time of the capsule endoscope is greater than a preset time threshold;
when the operation time is greater than the preset time threshold, determining whether the capsule endoscope reaches the preset detection position according to the value of the determination index;
when the operation time is not greater than the preset time threshold, determining whether the capsule endoscope reaches the preset detection position according to the value of the determination index after determining that the operation time of the capsule endoscope is greater than the preset time threshold.

9. The image-based position detection method of claim 1, wherein in the process of obtaining at least one image from each group of images, the method further comprises:
at least obtaining exposure parameters corresponding to the images for calculating the determination index;
and determining whether the capsule endoscope reaches the preset detection position according to the value of the determination index and the exposure parameters, wherein the exposure parameters are shutter time and image sensor gain participating in exposure in the process of obtaining the images.

10. The image-based position detection method of claim 9, further comprising:
configuring a counter;
obtaining the corresponding determination index immediately after each group of images is generated;
if the value of the counter N is greater than or equal to Nₘᵢₙ, determining that the capsule endoscope reaches the preset detection position;
wherein the value of Nₘᵢₙ is a positive integer greater than 1;
wherein the value of the counter is 0 in an initial state;
when the determination index is less than a preset first index threshold and greater than a preset second index threshold, and when time*gain is less than or equal to tg, accumulating the value of the counter by 1;
when the determination index is not greater than the preset second index threshold, and/or when time*gain is greater than tg, clearing the value of the counter to zero;
wherein, tg is a preset check value for exposure parameters, which is a constant;
if the number of the images obtained from each group of images is 1, "time" represents shutter time corresponding to the currently obtained image when exposed, and "gain" represents image sensor gain corresponding to the currently obtained image when exposed;
if the number of the images obtained from each group of images is at least 2, "time" represents a mean value of the shutter time corresponding to the images used for calculating the determination index in each group of images when exposed, and "gain" represents a mean value of the image sensor gain corresponding to the images used for calculating the determination index in each group of images when exposed.

11. An electronic device, comprising a memory and a processor, wherein the memory stores a computer program that runs on the processor, and the processor executes the program to implement steps of an image-based position detection method, wherein the image-based position detection method comprises:
driving a capsule endoscope to continuously capture a group of images at an interval of a first preset time t1, wherein each group of images comprises at least one image;
obtaining at least one image from each group of images, and calculating a determination index according to grayscale values of at least two channels of the image;
determining whether the capsule endoscope reaches a preset detection position according to a value of the determination index;
adjusting the capsule endoscope to a corresponding working mode according to a current position of the capsule endoscope when the capsule endoscope reaches the preset detection position; and
continue monitoring when the capsule endoscope has does not reach the preset detection position.

12. A computer-readable storage medium for storing a computer program, wherein the computer program is executed by the processor to implement steps of an image-based position detection method, wherein the image-based position detection method comprises:
driving a capsule endoscope to continuously capture a group of images at an interval of a first preset time t1, wherein each group of images comprises at least one image;
obtaining at least one image from each group of images, and calculating a determination index according to grayscale values of at least two channels of the image;
determining whether the capsule endoscope reaches a preset detection position according to a value of the determination index;
adjusting the capsule endoscope to a corresponding working mode according to a current position of the capsule endoscope when the capsule endoscope reaches the preset detection position; and
continue monitoring when the capsule endoscope has does not reach the preset detection position.
